# EUROPEAN PATENT SPECIFICATION

(11) **EP 4 303 227 B1**
(45) Date of publication and mention of the grant of the patent: **31.12.2025**
(21) Application number: 22763449.0
(22) Date of filing: 07.03.2022
(51) Int. Cl.: C07K 14/47, A61K 9/51, C12N 15/88, A61K 31/713, A61K 31/7088, A61K 38/00

(54) **METHOD FOR PRODUCING PROTEIN HAVING SUPRAMOLECULAR STRUCTURE IN WHICH BIOACTIVE SUBSTANCE IS ENCAPSULATED**
VERFAHREN ZUR HERSTELLUNG EINES PROTEINS MIT SUPRAMOLEKULARER STRUKTUR MIT DARIN VERKAPSELTER BIOAKTIVER SUBSTANZ
PROCÉDÉ DE PRODUCTION D'UNE PROTÉINE AYANT UNE STRUCTURE SUPRAMOLÉCULAIRE COMPRENANT UNE SUBSTANCE BIOACTIVE ENCAPSULÉE

(30) Priority: 05.03.2021 JP 2021035161
(43) Date of publication of application: 10.01.2024
(73) Proprietor: AJINOMOTO CO., INC., Chuo-ku Tokyo 104-8315 (JP)
(72) Inventor: NAKAHARA, Yuichi, Kawasaki-shi, Kanagawa 210-8681 (JP); ENDO, Yuta, Kawasaki-shi, Kanagawa 210-8681 (JP); INOUE, Ippei, Kawasaki-shi, Kanagawa 210-8681 (JP); OKASORA, Takahiro, Kawasaki-shi, Kanagawa 210-8681 (JP); HOSHIDA YAMAZAKI, Junko, Kawasaki-shi, Kanagawa 210-8681 (JP); KARAKAWA, Sachise, Kawasaki-shi, Kanagawa 210-8681 (JP)
(74) Representative: Mewburn Ellis LLP
(86) International application number: PCT/JP2022/009733
(87) International publication number: WO 2022/186399

(56) References cited:
- WO-A1-2020/075817
- WO-A1-2020/090708
- WO-A1-2020/095894
- CN-A- 106 110 333
- SANDRA MITIć, ET AL.: "Microsecond time-scale kinetics of transient biochemical reactions", PLOS ONE, vol. 12, no. 10, 8 October 2017 (2017-10-08), pages 1 - 15, XP055704704, DOI: 10.1371/journal.pone.0185888

## Description

### Technical Field

The present invention relates to a method for producing a protein having a supramolecular structure in which a bioactive substance is encapsulated.

### Background Art

Ferritin is a spherical shell protein having 24 subunits each of which is composed of a single polypeptide chain and which are self-organized and assembled through non-covalent bonds. Ferritin has an outer diameter of about 12 nm and has a cavity in a size of about 7 nm in the center. It is known that proteins having supramolecular structures, represented by ferritin, can be caused to contain medicines in the inside cavities. As the method for encapsulating a low-molecular medicine in ferritin, Non Patent Literature 1 discloses a method that adjusts the disassembling and the reassembling of ferritin by changing the pH of a solution, for example. Moreover, Patent Literature 1 proposes a method for encapsulating an organic compound in ferritin by means of a simple method of using a buffer solution having appropriate pH corresponding to the acid dissociation constant (pKa) of the organic compound, which is within such a pH range that does not destroy the structure of ferritin.

### Citation List

### Patent Literatures

Patent Literature 1: International Publication No. 2020/090708

### Non Patent Literature

Non Patent Literature 1: Journal of Controlled Release 196(2014) 184-196

### Summary of Invention

### Problems to be solved by the invention

Since the method of Patent Literature 1 is a method in which a low molecule actively enters the assembly through a space of the assembly, the size of a molecule which can be put inside the assembly is limited. Nucleic acid medicines such as siRNA and DNA, which have been attracting attention recent years, cannot be put inside the assembly through a space of the assembly. For this reason, a process of reassembling ferritin is required after disassembling ferritin and mixing the disassembly with a medicine. However, there is a possibility that when ferritin is reassembled, ferritin is aggregated, so that the quality as a medicine decreases. If aggregation occurs, the yield decreases, which in turn results in an increase in costs.

Hence, an object of the present invention is to provide a novel method for producing a protein having a supramolecular structure in which a bioactive substance is encapsulated.

### Means for solution of the problems

As a result of conducting earnest studies, the present inventors have found that when ferritin is reassembled by using a flow micro mixer ("FMM"), it is possible to suppress generation of misassemblies, and as a result, to obtain ferritin in which a bioactive substance is encapsulated, in a higher yield than a batch method. The present invention has been made based on such finding. Specifically, the present invention provides the following production method.
1. A method for producing a protein having a supramolecular structure in which a bioactive substance is encapsulated, comprising:
   (I) bringing a subunit of a protein, which forms a supramolecular structure, a bioactive substance, and a solution for forming the protein having the supramolecular structure from the subunit into contact with one another in a flow micro mixer.
2. The production method according to the above 1, wherein
   the protein having the supramolecular structure is ferritin.
3. The production method according to the above 1 or 2, wherein
   in the step (I), a sum of a flow rate of the subunit and a flow rate of the solution is about 10 mL/min or more.
4. The production method according to any one of the above 1 to 3, wherein
   the subunit is obtained by (II-1) changing pH of a solution containing the protein having the supramolecular structure to an acidic property or a basic property.
5. The production method according to the above 4, the solution used in the step (II-1) is a solution having pH of about 1.5 to about 3.0 or pH of about 10 to 12.
6. The production method according to any one of the above 1 to 5, wherein
   the solution used in the step (I) for forming the protein having the supramolecular structure from the subunit is a solution having pH of about 5.0 to about 9.0.
7. The production method according to any one of the above 1 to 3, wherein
   the subunit is obtained by (II-2) adding a solvent to a solution containing the protein having the supramolecular structure.
8. The production method according to the above 7, wherein
   the solvent used in the step (II-2) is selected from the group consisting of dimethyl sulfoxide (DMSO), N,N-dimethylformamide (DMF), acetonitrile, and ethanol.
9. The production method according to the above 7 or 8, wherein
   the solution used in the step (I) is selected from the group consisting of a Tris buffer solution, a HEPES buffer solution, a phosphate buffer solution, a boric acid buffer solution, a citric acid buffer solution, a carbonic acid buffer solution, a glycine buffer solution, and the like.
10. The production method according to any one of the above 4 to 9, wherein
   the step (II-1) or (II-2) is conducted by using a flow micro mixer.
11. The method according to any one of the above 1 to 10, wherein
   the bioactive substance is selected from the group consisting of siRNAs, DNAs, oligopeptides each having a mass average molecular weight of about 1,000 to about 20,000, or combinations of these.

### Advantageous Effects of Invention

The present invention makes it possible to suppress generation of misassemblies. In addition, since the present invention makes it possible to shorten a time for which a bioactive substance is exposed to an acid or a base or a solvent used for disassembling, it is possible to suppress denaturation or decomposition of the bioactive substance. As a result, the present invention makes it possible to obtain a protein having a supramolecular structure in which a bioactive substance is encapsulated, in a high yield.

### Brief Description of Drawings

Fig. 1 is a graph showing misassembly proportions when ferritin was disassembled and reassembled by a batch method.
Fig. 2 is a graph showing misassembly proportions relative to the sum of flow rates at the time of reassembling when ferritin was disassembled and reassembled by using a FMM. The legends indicate ferritin concentrations of disassembled solutions.
Fig. 3 is a graph showing assembly proportions when disassembling-reassembling was conducted by using combinations of Batch-Batch, FMM-Batch, Batch-FMM, and FMM-FMM.

### Description of Embodiments

### 1. Definitions

The terms used in the present specification are defined as follows.

The term "supramolecule" means an assembly having a high-order structure in which multiple molecules or ions are assembled by interaction like non-covalent bonds (for example, by self-organization).

The term "disassembling" means dissociating a protein having a supramolecular structure into individual subunits.

The term "reassembling" means causing a disassembled protein to take a supramolecular structure again.

The term "pH" indicates a value measured by using a glass electrode at 25°C.

The term "misassembly" collectively refers to, as explained in terms of ferritin as an example, proteins in which 24 subunits do not form an assembly, such as those in which two 24-mers are bound through divalent metal ions or the like, assemblies having less than 24 subunits, assemblies having more than 24 subunits, lumps of proteins aggregated without being assembled, and the like, although ferritin correctively assembled is an assembly of 24 subunits.

### 2. Production Method of the Present Invention

The present invention is a method for producing a protein taking a supramolecular structure in which a bioactive substance is encapsulated in a cavity present in the center thereof by assembling subunits of the protein taking the supramolecular structure in the presence of the bioactive substance in a FMM.

A step of obtaining a bioactive substance-encapsulated assembly from a subunit, that is, a reassembling step is referred to as a step (I).

A FMM is generally a device that mixes two or more liquids in a space of several tens or several hundreds of microns. The FMM used in the method of the present invention is of a continuous flow type.

The FMM used in the present invention has, for example, a first flow path through which subunits flow, a second flow path through which a bioactive substance flows, and a third flow path through which a solution ("reassembling solution") for forming an assembly from the subunits, and includes a place where the subunits, the bioactive substance, and the solution merge and are mixed. It is also possible to mix the flow of the subunits and the flow of the bioactive substance in advance, and then to merge and mix the flow of the reassembling solution in the mixture, or to simultaneously merge and mix the flow of the subunits, the flow of the bioactive substance, and the flow of the solution at a mixing place. In the case where the bioactive substance to be encapsulated is weak to an acid, a base, or a solvent used for disassembling, the latter is preferable because the time for which the bioactive substance comes into contact with the acid or the like can be shortened.

### [Reassembling]

The means for reassembling a protein having a supramolecular structure from subunits is not particularly limited. For example, the reassembling can be achieved by (I-1) adjusting the pH of the mixed flow of the flow of the subunits and the flow of the bioactive substance to neutral, for example, about 5 to about 9. This step is suitable when the disassembling is conducted in a step (II-1) described later. The pH adjustment in the step (I-1) can be achieved by adding a reassembling solution to the mixed flow. The pH of the reassembling solution can be set as appropriate depending on the pH of the disassembling solution, but may be preferably around 5.0 to 10.0 in general. The substance for making the pH neutral includes a Tris buffer solution, a HEPES buffer solution, a phosphate buffer solution, a boric acid buffer solution, a citric acid buffer solution, a carbonic acid buffer solution, a glycine buffer solution, and the like. Among these, a Tris buffer solution, a phosphate buffer solution, and the like are preferable since they have wide ranges of pH-buffering abilities. A solution containing a substance for making the pH neutral is desirably a solution selected from the group consisting of phosphoric acid or Tris hydrochloride each having pH of about 5.0 to about 9.0.

In the case where the protein is ferritin, it is known that the ferritin is reassembled when the pH is adjusted to 5.0 (Biochemistry 1987, 26, 1831-1837). Hence, the pH of the reassembling solution is preferably about 5.0 to about 9.0, and more preferably about 7.0 to about 9.0. The substance for making the pH acidic is preferably hydrochloric acid because it is desirably a low molecule in order to avoid competition with the substance to be encapsulated.

The reassembling also can be achieved by (I-2) diluting the mixed flow of the flow of the subunits and the flow of the bioactive substance. This step is suitable when the disassembling is conducted in a step (II-2) described later. In the case where the assembly protein is dissociated into subunits by adding a solvent to the assembly protein, the presence of the solvent prevents the subunits from naturally reassembling. In view of this, the reassembling is made possible by removing the solvent with dilution. Hence, it is favorable to adjust the volume of each solution such that the dilution factor is, for example, 10 to 20 times, which varies depending on the concentration or the flow rate of the subunits though. The dilution in the step (I-2) can be conducted by adding a diluent solution as the reassembling solution to the mixed flow. The diluent solution includes a Tris buffer solution, a HEPES buffer solution, a phosphate buffer solution, a boric acid buffer solution, a citric acid buffer solution, a carbonic acid buffer solution, a glycine buffer solution, and the like. Particularly, a Tris buffer solution is preferable because it has a high buffering ability in a range from neutral to weak alkalinity. In addition, the dilution can be conducted by dialysis. Specifically, for example, a ferritin solution disassembled by using an acid is put in a dialysis tube, and dialysis is conducted by using an external solution as a diluent solution to make the pH in the dialysis tube neutral, thus achieving reassembling.

Although the reassembling means can be selected as appropriate by a person skilled in the art depending on the concentration of the supramolecular structure reassembled and the amount of the solution, the step (I-1) is preferable because a stable supramolecular structure can be efficiently obtained.

In the present invention, it is possible to shorten the time required for forming an assembly by using a FMM. As a result, the generation of misassemblies can be suppressed. When the flow rate of the subunit solution and the flow rate of the reassembling solution are set to, for example, about 10 mL/min or more, misassemblies decrease. When both flow rates are independently about 10 to about 100 mL/min, misassemblies further decrease. Particularly, when the sum of the flow rate of the subunit solution and the flow rate of the reassembling solution (hereinafter also referred to as "TFR" (the abbreviation of Total Flow Rate) is larger, the misassembly proportion decreases. In particular, a TFR of about 10 mL/min or more is industrially advantageous because the misassembly proportion can be suppressed low even when the scale is increased. Particularly, it is preferable that the flow rate of the subunit solution be larger than the flow rate of the reassembling solution. In this case, when the difference between the flow rate of the subunit solution and the flow rate of the reassembling solution is around 1 to 10, the mixing performance is enhanced, so that the misassembly proportion can be suppressed.

As the flow micro mixer used in the present invention, a general one such as a slit type, a disk type, or a forced contact type can be used. A forced contact type is preferable because it is possible to minimize a clogging trouble due to a flow path, and achieve an excellent mixing performance.

The sectional shape of the flow path is not particularly limited; however, using one having a V shape can reduce misassemblies.

The inner diameter of the flow path is not particularly limited; however, for example, one having an inner diameter of about 0.1 to about 1.0 mm can be favorably used. When the inner diameter is about 0.2 to about 0.5 mm, it is further advantageous in reducing the misassembly proportion.

Using a flow micro mixer having a V shape and including a flow path having an inner diameter of about 0.2 to about 0.5 µm can further reduce misassemblies.

The length of each flow path can be set as appropriate, but is preferably about 10 to about 20000 µm, further preferably about 100 to about 5000 µm, and preferably about 200 to about 3000 µm because a sufficient mixing performance can be obtained. Note that the lengths of the respective flow paths may be equal to or different from each other, but are preferably equal.

As the material of the flow path, for example, an inorganic material such as SUS or an organic material such as polytetrafluoroethylene (PTFE) can be used.

As the flow micro mixer used in the present invention, for example, those commercially available from Fraunhofer IMM, YMC Co., Ltd., and Sankoh Seiki Kogyo Co., Ltd. can be used.

The mixture liquid which has flowed out of the flow micro mixer is sent to a receiver tank. A protein having a supramolecular structure formed again and containing a bioactive substance in the center portion can be recovered from the receiver tank by means of an ultrafiltration or column chromatography technique.

A protein which can be obtained by the production method of the present invention is an assembly having a supramolecular structure, and has a cavity in which a bioactive substance can be contained, in the center. Such a protein includes, for example, ferritin, an 11-mer protein called TRAP, bromoperoxidase, a matrix protein of M1 virus, galactoside O-acetyltransferase, and the like. One of the proteins may be used alone or two or more of them may be used in combination. The subunits of ferritin include an H subunit and an L subunit. In the present invention, one of these may be used or both of these may be used in combination. Among these, ferritin is preferable because disassembling and reassembling can be easily controlled by adjusting pH.

The subunit used in the step (I) is composed of a single polypeptide chain forming such an assembly protein. The subunit is dissolved in an appropriate solvent and used in the state of a solution. The solvent for this includes dimethyl sulfoxide (DMSO), N,N-dimethylformamide (DMF), acetonitrile, ethanol, and the like. Among these, DMSO and DMF, each of which having an amphiphilic property, are preferable. The concentration of the subunit at this time is preferably about 0.1 to about 50 g/L because a solution having a low viscosity can be easily handled.

### [Disassembling]

The production method of the present invention may include the step of disassembling the protein having the supramolecular structure before the step (I). That is, the production method of the present invention may include the step of dissociating the protein in the state of an assembly into individual subunits. This step is referred to as a step (II). The subunits obtained in this step can be used in the step (I). According to the finding of the present inventors, it is considered that the effect which the time required for disassembling imposes on the yield of the assembly after the reassembling is not greater than the effect which the time required for reassembling imposes on the yield of the assembly after the reassembling. Hence, the disassembling may be conducted through batch or flow.

The disassembling means includes, for example, making acidic the pH of the solution containing the protein assemblies before the bioactive substance is encapsulated. This step is hereinafter referred to as a step (II-1). The pH at this time may be around 1.5 to 3.0. A substance for making the pH acidic includes hydrochloric acid, glycine hydrochloride, sulfuric acid, and the like. Among these, a glycine hydrochloride buffer solution is preferable because it is excellent in controlling the pH. As a solution containing a substance for making the pH acidic, those selected from the group consisting of hydrochlorides having pH of about 1 to about 3.0 are excellent in control.

It is known that in the case where the protein is ferritin, ferritin is disassembled by adjusting the pH to 2.5 (Biochemistry 1987, 26, 1831-1837). Hence, the pH of the solution is preferably about 1.5 to about 3.0, and more preferably about 1.5 to about 2.5. As the substance for making the pH acidic, glycine hydrochloride, which is excellent in controlling pH, is preferable.

It is reported that ferritin can be reversibly decomposed with pH of about 10 to 12 (Yi Gou, et. al., Frontiers in Pharmacology, 2018 Apr 27; 9:421). Hence, in the case where the protein is ferritin, ferritin can also be disassembled into its subunits by adjusting the pH of the solution containing the protein assemblies before the bioactive substance is encapsulated to about 10 to 12 in the step (II-1).

Since empirically, an acidic property is more advantageous than an alkaline property in terms of disassembling, it is preferable to disassemble the protein by using a solution having pH of about 1.5 to 3.0.

The disassembling means also includes adding a solvent to a solution containing assemblies. This step is hereinafter referred to as a step (II-2). The solvent which can be used in the step (II-2) includes DMSO, DMF, acetonitrile, ethanol, and the like. In the case where the protein is ferritin, DMF or DMSO is preferable from the viewpoint of maintaining the ferritin subunit structure.

Although the disassembling means can be selected by a person skilled in the art depending on the concentration of ferritin and the scale of conducting disassembling, the step (II-1) is preferable, and a method for making the pH acidic is more preferable, in consideration of the work of removing the solvent and the effect of the remaining solvent.

When the step (II-1) is employed as the disassembling means, the reassembling step is preferably (I-1). When the step (II-2) is employed as the disassembling means, the reassembling step is preferably (I-2). The misassemblies can be further reduced by employing both the step (II-1) and the step (I-1).

In the case where the disassembling is conducted through flow, the disassembling can be conducted by using a flow micro mixer (FMM). In this case, a solution containing the protein assemblies in which the bioactive substance is not encapsulated, and a solution containing a substance for making the pH acidic and/or a diluent solution of a solvent are supplied from separate flow paths, respectively. The flow rates of the former and latter in this case may be equal or different, and may independently be about 1 mL/min or more. When the flow rates are about 5 to about 100 mL/min, it can be expected that the efficiency of encapsulation is further enhanced. When both flow rates are about 5 mL/min, the efficiency of encapsulation is enhanced.

It is preferable to conduct the disassembling as well by using a FMM because misassemblies can be reduced. In this aspect, when the disassembling means is the step (II-1) and the reassembling means is the step (I-1), misassemblies can be further reduced. Particularly, the flow rates of the solution containing the protein assemblies in which a bioactive substance is not encapsulated and the solution containing the substance for making the pH acidic are independently preferably about 1 mL/min or more, more preferably about 5 to about 100 mL/min, and most preferably about 5 mL/min, from the viewpoint of improving the encapsulation efficiency. In particular, in the above aspect, the flow rate of the subunit solution and the flow rate of the reassembling solution are independently preferably about 10 to about 100 mL/min because misassembly are further reduced, and the TFR is more preferably about 10 mL/min or more. The difference between the flow rate of the subunit solution and the flow rate of the reassembling solution is particularly preferably around 1 to 10 because the mixing performance can be enhanced, so that the misassembly proportion can be suppressed.

### [Bioactive Substance]

The bioactive substance to be encapsulated includes substances each having a mass average molecular weight of, for example, around 1,000 to 20,000, such as siRNAs (small interfering RNAs), DNAs, and oligopeptides. Among these, since the size of the cavity inside ferritin is around 7 nm, the mass average molecular weight of the bioactive substance is more preferably around 1,000 to 15,000, and further preferably around 1,000 to 10,000.

Since the size of the cavity varies depending on the assembly, the bioactive substance suitable to encapsulate is different. For example, since the size of the cavity of ferritin is about 7 nm, peptides, DNAs, and siRNAs are suitable.

The bioactive substance is brought into contact with the protein, in the state of a solution, and encapsulated in the protein. The solvent for forming the solution of the bioactive substance includes a Tris buffer solution and the like. Among these, the solvent is preferably neutral in order to suppress hydrolysis by an acid. The concentration of the solution of the bioactive substance is preferably about 1 to about 100 µM because the aggregation of the bioactive substance can be suppressed.

The protein assemblies in which the bioactive substance is encapsulated and the bioactive substance which is not encapsulated in the protein assemblies in the reassembling step can be recovered by using an ultrafiltration membrane or a dialysis membrane, for example, a tangential flow dialysis of Spectrum Labs. The bioactive substance thus recovered can be reused. For example, the encapsulation of the bioactive substance into the protein assemblies can also be continuously conducted by merging the solution containing the recovered bioactive substance into the flow of the subunit solution in the step (I).

In the case where the disassembling is conducted by using a FMM, the encapsulation of the bioactive substance into the protein assemblies can also be continuously conducted by merging the solution containing the recovered bioactive substance into the flow of the subunit solution in the step (I), or the flow of the solution having pH of about 1.5 to about 3.0 or the solution having pH of around 10 to 12 in the step (II-1), or the flow of the solvent or the flow of the solution of the protein assemblies which do not contain the bioactive substance in the step (II-2). In the case where the bioactive substance to be encapsulated is weak to an acid, a base, or a solvent used in the disassembling, it is preferable to merge the solution containing the recovered bioactive substance into the flow of the subunit solution in the step (I), or the flow of the solution of the protein assemblies which do not contain the bioactive substance because the time for which the bioactive substance comes into contact with the acid and the like can be shortened.

In a particularly preferable aspect of the present invention, the step (II-1) is conducted by adjusting the pH of the solution of the protein assemblies in which the bioactive substance is not encapsulated, to about about 2.3 to about 2.5 by using a glycine hydrochloride buffer having pH of about 2 to about 2.5, and the step (I-1) is conducted by making the pH of the subunit solution of the disassembled protein neutral by using a Tris hydrochloride buffer having pH of about 7.0 to about 9.0. In this way, the reassembling can be conducted. In this case, when the sum of the flow rate of the subunit solution and the flow rate of the Tris hydrochloride buffer is about 10 mL/min or more, the misassembly proportion can be reduced. Further particularly, it is desirable that the protein be ferritin and the bioactive substance be DNA.

The method of the present invention can be conducted without using a bioactive substance. That is, it is possible to disassemble and reassemble a protein taking a supramolecular structure by the present invention. This method makes it possible to use a supramolecular structure as an adsorption carrier and the like.

### Examples

### <Analysis Conditions>

### [Method for Analyzing Misassembly Proportion]

The misassembly proportion was analyzed by using gel filtration chromatography under the following conditions.

| Column | SUPERDEX 200 INCREASE 10/300GL manufactured by GE HealthCare Japan | [-] |
|---|---|---|
| Eluent | PBS buffer (pH 7.4) | [-] |
| Flow rate | 0.8 | [mL/min] |
| Column temperature | 25 | [°C] |
| Detection wavelength | 260 | [nm] |

### [Method for Analyzing Amount of Encapsulated DNA]

The amount of encapsulated DNA contained inside ferritin was measured by quantifying the P concentration in the amount of DNA contained inside ferritin by using HPLC-ICP-MS. Note that HPLC is the abbreviation of high-performance liquid chromatography and ICP-MS is the abbreviation of inductively coupled plasma mass spectrometer. Each analysis condition is shown below.

**Table A**

| [HPLC analysis conditions] | |
|---|---|
| Apparatus | Ultimate 3000 manufactured by Thermo |
| Separation column | Superdex 200 Increase (inner diameter: 3.2 mm; length: 300 mm) manufactured by Cytiva (the former GE HealthCare) |
| Eluent | 200 mM Ammonium acetate aqueous solution |
| Column temperature | 26°C |
| Flow rate | 0.1 mL/min |
| Elution | Isocratic elution |
| Injection volume [ICP-MS/MS analysis conditions] | 5 µL |
| Apparatus | 8900 IPC-MS Triple Quad manufactured by Agilent Technologies |
| RF | 1550 W |
| Carrier gas | 1.05 L/min |
| Spray chamber temperature | 2°C |
| Sampling position | 10 mm |
| Detection mode | MS/MS |
| Cell gas | O2 |
| Detection channel (m/z) | P: 31->47, S: 32->48 |

### <Method for Analyzing Amounts of Encapsulated siRNA and Peptide>

To ferritin recovered by the same method in the method for analyzing a misassembly proportion, a 0.2N hydrochloric acid (HCl) solution was added dropwise to adjust the pH to 2 or less to disassemble ferritin again. The disassembled solution was subjected to gel filtration chromatography again to quantify the encapsulated component.

A solution b used below was a 100 mM glycine hydrochloride buffer prepared by diluting a 1000 mM glycine hydrochloride buffer (pH 2.3) with ultra-pure water.

A solution d was a 350 mM Tris hydrochloride buffer prepared by diluting a 1000 mM Tris hydrochloride buffer (pH 9.0) with ultra-pure water.

Note that as ferritin, ferritin (lot A) which we fermented and purified in accordance with the method disclosed in Patent Literature 1 was used.

### 1. Comparison of Aggregate Proportion by FMR/Batch

### Reference Examples 1 to 7 (Study on Reassembling Flow Rate of FMM with 1 g/L of Ferritin)

Ferritin was disassembled by mixing a 10 mM Tris buffer solution a containing 2 g/L of ferritin with the solution b to adjust the pH to about 2.3 to 2.5 by using a V-shaped mixer having an inner diameter of 500 µm. The flow rate of each solution was set to 5 mL/min. In this way, a disassembled solution c having a ferritin concentration of 1 g/L and a glycine hydrochloride concentration of 50 mM was obtained.

Ferritin was reassembled by mixing the disassembled solution c with the solution d at predetermined flow rates to adjust the pH to about 7.3 by using the V-shaped mixer having an inner diameter of 500 µm. The solution thus obtained was analyzed by the above method and conditions to measure the misassembly proportion. The flow rates of the solution c and the solution d as well as the misassembly proportions are shown in Table 1.

**Table 1**

| | Ferritin disassembled solution c | 350 mM Tris hydrochloride buffer solution d | Misassembly proportion |
|---|---|---|---|
| | [mL/min] | [mL/min] | [%] |
| Reference Example 1 | 0.4 | 0.1 | 17.3 |
| Reference Example 2 | 0.8 | 0.2 | 12.6 |
| Reference Example 3 | 2 | 0.5 | 11.3 |
| Reference Example 4 | 4 | 1 | 10.5 |
| Reference Example 5 | 8 | 2 | 11.7 |
| Reference Example 6 | 20 | 5 | 11.6 |
| Reference Example 7 | 36 | 9 | 11.0 |

### Reference Examples 8 to 11 (Study on Reassembling Flow Rate of FMM with 3 g/L of ferritin)

Ferritin was disassembled and reassembled in the same manner as in Reference Examples 1 to 7 except that the ferritin concentration of the solution a was changed to 6 g/L. Note that the ferritin concentration in the disassembled solution c was 3 g/L. The flow rates of the solution c and the solution d as well as the misassembly proportions are shown in Table 2.

**Table 2**

| | Ferritin disassembled solution c | 350 mM Tris hydrochloride buffer solution d | Misassembly proportion |
|---|---|---|---|
| | [mL/min] | [mL/min] | [%] |
| Reference Example 8 | 0.4 | 0.1 | 23.2 |
| Reference Example 9 | 8 | 2 | 20.5 |
| Reference Example 10 | 20 | 5 | 19.9 |
| Reference Example 11 | 36 | 9 | 20.8 |

### Reference Examples 12 to 14 (Study on Reassembling Flow Rate of FMM with 5 g/L of ferritin)

Ferritin was disassembled and reassembled in the same manner as in Reference Examples 1 to 7 except that the ferritin concentration of the solution a was changed to 10 g/L. Note that the ferritin concentration in the disassembled solution c was 5 g/L. The flow rates of the solution c and the solution d as well as the misassembly proportions are shown in Table 3.

**Table 3**

| | Ferritin disassembled solution c | 350 mM Tris hydrochloride buffer solution d | Misassembly proportion |
|---|---|---|---|
| | [mL/min] | [mL/min] | [%] |
| Reference Example 12 | 0.4 | 0.1 | 35.9 |
| Reference Example 12 | 8 | 2 | 31.2 |
| Reference Example 14 | 20 | 5 | 32.6 |

### Reference Example 15 (Batch for Comparison, disassembled solution c=1 g/L of ferritin, 200 µL)

Ferritin was disassembled by a batch method in which 100 µL of a 10 mM Tris buffer solution a containing 2 g/L of ferritin was placed in an Eppendorf tube, and 100 µL of the solution b was added and mixed thereto to adjust the pH to about 2.3 to 2.5. In this way, a disassembled solution c having a ferritin concentration of 1 g/L was obtained. Note that unless otherwise stated, in the columns of Examples, the final concentration of the disassembled solution c was made equal between the batch method and the flow micro mixer method.

Ferritin was reassembled by a batch method in which 200 µL of the disassembled solution c was placed in an Eppendorf tube, and 50 µL of the solution d was added and mixed thereto to adjust the pH to about 7.3. As a result, the misassembly proportion was 10.1%.

### Reference Example 16 (Batch for Comparison, disassembled solution c=5 g/L of ferritin, 2 mL)

Ferritin was disassembled by a batch method in which 1 mL of a 10 mM Tris buffer solution a containing 10 g/L of ferritin was placed in an Eppendorf tube, and 1 mL of the solution b was added and mixed thereto to adjust the pH to about 2.3 to 2.5. In this way, a disassembled solution c having a ferritin concentration of 5 g/L was obtained.

Ferritin was reassembled by a batch method in which 2 mL of the disassembled solution c was placed in a Falcon tube, and 500 µL of the solution d was added and mixed thereto to adjust the pH to about 7.3. The misassembly proportion at this time was 31.0%.

### Reference Example 17 (Batch for Comparison, disassembled solution c=5 g/L of ferritin, 10 mL)

Ferritin was disassembled by a batch method in which 5 mL of a 10 mM Tris buffer solution a containing 10 g/L of ferritin was placed in a beaker, and 5 mL of the solution b was added thereto, followed by stirring with a magnetic stirrer to adjust the pH to about 2.3 to 2.5. In this way, a disassembled solution c having a ferritin concentration of 5 g/L was obtained.

Ferritin was reassembled by a batch method in which 2 mL of the solution d was added to a beaker containing 10 mL of the disassembled solution c, followed by stirring with a magnetic stirrer to adjust the pH to about 7.3. The misassembly proportion at this time was 33.9%.

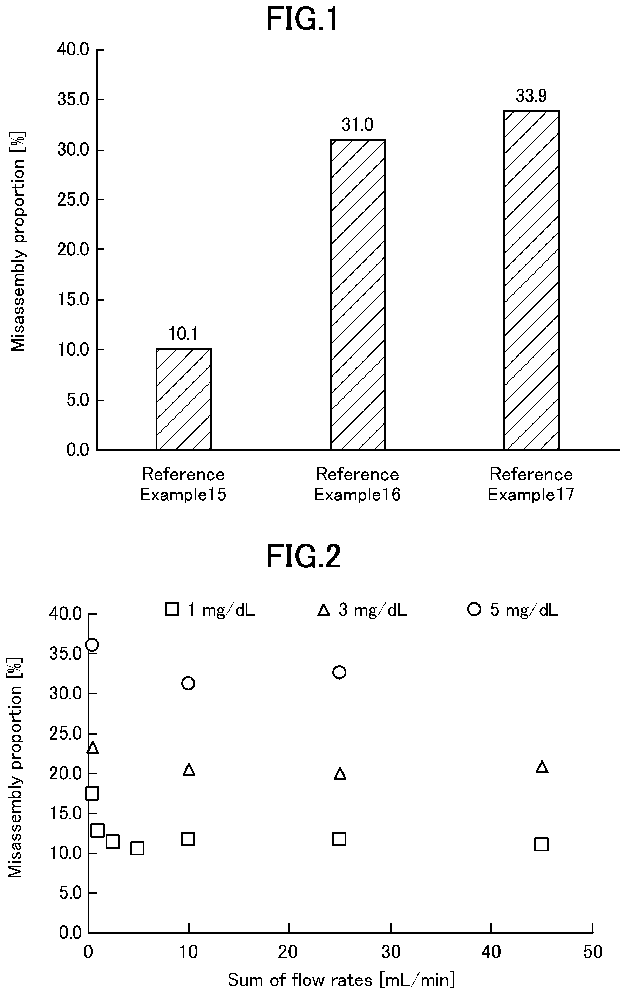

The above Reference Examples 15 to 17 for comparison may be summarized as shown in Fig. 1. By comparing Reference Examples 15 and 16, it is obvious that misassemblies tend to increase in accordance with the ferritin concentration. In addition, by comparing Reference Examples 16 and 17, it is obvious that misassemblies tend to increase as the amount of the solution at the reassembling is larger. This is because as the amount of the solution increases, a longer time is required until the complete mixing, and Kouhei Tsumoto et al, Protein Expression and Purification, 28 (2003) 1-8 also describes similar cases (particularly, the sections of "Dilution" and "Mixing").

On the other hand, regarding Reference Examples 1 to 14 using the FMM, Fig. 2 is obtained by plotting the sum of the flow rates at the reassembling on the horizontal axis and the misassembly proportion on the vertical axis. In the range with small sums of the flow rates, the misassembly proportion is large. As the flow rates increase, the misassembly proportion decreases. When the sum of the flow rates is 10 mL/min or more, the misassembly proportion becomes constant. That is, in the batch method, the misassembly proportion increase as the amount of the solution increases. In the case of using the FMM, the misassembly proportion does not increase even when the sum of the flow rates is increased, and further, it is possible to obtain a misassembly proportion similar to that in the case where the method was conducted in a small scale like Examples 15 and 16.

### Reference Examples 18 to 19 (Check of FMM Reproducibility)

The same experiments as in Reference Example 5 were further conducted twice, and the misassembly proportions were measured to be 11.3% and 9.2%, respectively. From this, the FMM is also excellent in reproducibility, and is superior to the batch method in terms of the production process as well.

### Reference Example 20 (Batch Method)

Ferritin was disassembled by a batch method in which 1 mL of a 10 mM Tris buffer solution a containing 2 g/L of ferritin was placed in an Eppendorf tube, and 1 mL of the solution b was added and mixed thereto to adjust the pH to about 2.3 to 2.5. In this way, a disassembled solution c having a ferritin concentration of 1 g/L was obtained.

First, 2 mL of the disassembled solution c was placed in a Falcon tube, and 500 µL of the solution d was gently added thereto to gradually adjust the pH to about 7.3. In this way, ferritin was reassembled by a batch method. The misassembly proportion was measured to be 67.1%. It is thus obvious that a gradual pH adjustment, that is, the speed of mixing affects an increase in misassembly proportion.

### 2. Cross Test in Reassembling/Disassembling using FMM

In Reference Examples 21 to 24 disclosed below, ferritin of a lot (lot B) different from that in Reference Examples 1 to 20 which was newly prepared in accordance with the method described in Patent Literature 1 was used.

### Reference Example 21 (Batch for Comparison, 1 g/L of ferritin, 200 µL); One Different in Lot from Reference Example 15

Ferritin was disassembled by a batch method in which 100 µL of a 10 mM Tris buffer solution a containing 2 g/L of ferritin was placed in an Eppendorf tube, and 100 µL of the solution b was added and mixed thereto to adjust the pH to about 2.3 to 2.5. A disassembled solution c having a ferritin concentration of 1 g/L was obtained.

Ferritin was reassembled by a batch method in which 200 µL of the disassembled solution was placed in an Eppendorf tube, and 50 µL of the solution d was added thereto to adjust the pH to about 7.3. As a result, the misassembly proportion was 3.6%.

### Reference Examples 22 to 24 (Study on Reassembling Flow Rate of FMM with 1 g/L of Ferritin); Those Different in Lot from Reference Examples 3 to 5

Ferritin was disassembled by mixing a 10 mM Tris buffer solution a containing 2 g/L of ferritin with the solution b to adjust the pH to about 2.3 to 2.5 by using the V-shaped mixer having an inner diameter of 500 µm. The flow rate of each solution was set to 5 mL/min. In this way, a disassembled solution c having ferritin of 1 g/L and glycine hydrochloride of 50 mM was obtained.

Ferritin was reassembled by mixing the disassembled solution c with the solution d at predetermined flow rates to adjust the pH to about 7.3 by using the V-shaped mixer having an inner diameter of 500 µm.

The flow rates of the solution c and the solution d as well as the misassembly proportions are shown in Table 4.

**Table 4**

| | Ferritin disassembled solution c | 350 mM Tris hydrochloride buffer solution d | Misassembly proportion |
|---|---|---|---|
| | [mL/min] | [mL/min] | [%] |
| Reference Example 22 | 2 | 0.5 | 5.3 |
| Reference Example 23 | 4 | 1 | 5.1 |
| Reference Example 24 | 8 | 2 | 4.2 |

In Reference Examples 25 to 28 disclosed below, ferritin (lot C) which was prepared separately from those in Reference Examples 1 to 24 in accordance with the method described in Patent Literature 1 was used.

### Reference Example 25 (Batch for Comparison, 1 g/L of ferritin, 2 mL)

First, 1000 µL of a 10 mM Tris buffer solution a containing 2 g/L of ferritin was placed in an Eppendorf tube, and 1000 µL of the solution b was added and mixed thereto to adjust the pH to about 2.3 to 2.5. As a result, a disassembled solution c having a ferritin concentration of 1 g/L was obtained. In this way, ferritin was disassembled by a batch method.

Ferritin was reassembled by a batch method in which 2000 µL of the disassembled solution c was placed in an Eppendorf tube, and 500 µL of the solution d was added thereto to adjust the pH to about 7.3. As a result, the misassembly proportion was 17.0%.

### Reference Example 26 (Example in which Disassembling-Reassembling were conducted by FMM-Batch (1 g/L))

Ferritin was disassembled by mixing a 10 mM Tris buffer solution a containing 2 g/L of ferritin with the solution b to adjust the pH to about 2.3 to 2.5 by using the V-shaped mixer having an inner diameter of 500 µm. The flow rate of each solution was set to 5 mL/min. In this way, a disassembled solution c having ferritin of 1 g/L and glycine hydrochloride of 50 mM was obtained.

Ferritin was reassembled by a batch method in which 2000 µL of the disassembled solution c was placed in an Eppendorf tube, and 500 µL of the solution d was added thereto to adjust the pH to about 7.3. As a result, the misassembly proportion was 11.2%.

### Reference Example 27 (Example in which Disassembling-Reassembling were conducted by Batch-FMM (1 g/L))

Ferritin was disassembled by a batch method in which 2000 µL of a 10 mM Tris buffer solution a containing 2 g/L of ferritin was placed in an Eppendorf tube, and 2000 µL of the solution b was mixed therewith to adjust the pH to about 2.3 to 2.5. A disassembled solution c having a ferritin concentration of 1 g/L was obtained.

Ferritin was reassembled by mixing the disassembled solution c and the solution d to adjust the pH to about 7.3 by using the V-shaped mixer. At this time, the flow rate of the disassembled solution c was set to 8 mL/min, and the flow rate of the solution d was set to 2 mL/min. As a result, the misassembly proportion was 8.0%.

### Reference Example 28 (Example in which Disassembling-Reassembling were Conducted by FMM-FMM (1 g/L))

Ferritin was disassembled by mixing a 10 mM Tris buffer solution a containing 2 g/L of ferritin and the solution b to adjust the pH to about 2.3 to 2.5 by using the V-shaped mixer having an inner diameter of 500 µm. The flow rate of each solution was set to 5 mL/min. In this way, a disassembled solution c having ferritin of 1 g/L and glycine hydrochloride of 50 mM was obtained.

Ferritin was reassembled by mixing the disassembled solution c and the solution d to adjust the pH to about 7.3 by using the V-shaped mixer. At this time, the flow rate of the disassembled solution c was set to 8 mL/min, and the flow rate of the solution d was set to 2 mL/min. As a result, the misassembly proportion was 6.3%.

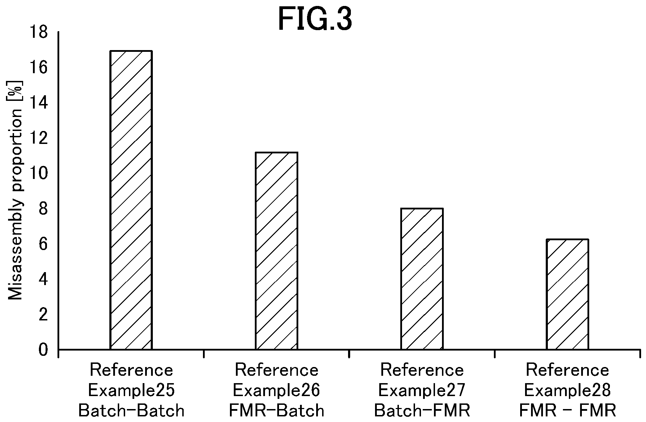

Relations as shown in Fig. 3 are obtained by extracting and summarizing the results of Reference Examples 25 to 28.

A list of the conditions of Reference Examples 1 to 28 is shown in Table 5.

**Table 5**

| Fig. No. | Lot | Ref. Ex. No. | Disassembling | | | | Reassembling | | | | Misassembly proportion [%] |
|---|---|---|---|---|---|---|---|---|---|---|---|
| | | | Reaction method | Flow rate [mL/min] | | Conc. after disassemblin g [g/L] | Reaction method | Flow rate [mL/min] | | Conc. after merging [g/L] | |
| | | | | Solution a | Solution b | | | Disassembled solution c | Solution d | | |
| 2 | A | 1 | FMM | 5 | 5 | 1 | FMM | 0.4 | 0.1 | 0.8 | 17.3 |
| | A | 2 | FMM | 5 | 5 | 1 | FMM | 0.8 | 0.2 | 0.8 | 12.6 |
| | A | 3 | FMM | 5 | 5 | 1 | FMM | 2 | 0.5 | 0.8 | 11.3 |
| | A | 4 | FMM | 5 | 5 | 1 | FMM | 4 | 1 | 0.8 | 10.5 |
| | A | 5 | FMM | 5 | 5 | 1 | FMM | 8 | 2 | 0.8 | 11.7 |
| | A | 6 | FMM | 5 | 5 | 1 | FMM | 20 | 5 | 0.8 | 11.6 |
| | A | 7 | FMM | 5 | 5 | 1 | FMM | 36 | 9 | 0.8 | 11.0 |
| | A | 8 | FMM | 5 | 5 | 3 | FMM | 0.4 | 0.1 | 2.4 | 23.2 |
| | A | 9 | FMM | 5 | 5 | 3 | FMM | 8 | 2 | 2.4 | 20.5 |
| | A | 10 | FMM | 5 | 5 | 3 | FMM | 20 | 5 | 2.4 | 19.9 |
| | A | 11 | FMM | 5 | 5 | 3 | FMM | 36 | 9 | 2.4 | 20.8 |
| | A | 12 | FMM | 5 | 5 | 5 | FMM | 0.4 | 0.1 | 4 | 35.9 |
| | A | 13 | FMM | 5 | 5 | 5 | FMM | 8 | 2 | 4 | 31.2 |
| | A | 14 | FMM | 5 | 5 | 5 | FMM | 20 | 5 | 4 | 32.6 |
| 1 | A | 15 | Batch | 0.1 | 0.1 | 1 | Batch | 0.05 | 0.2 | 0.2 | 10.1 |
| | A | 16 | Batch | 1 | 1 | 5 | Batch | 0.5 | 2 | 1 | 31.0 |
| | A | 17 | Batch | 5 | 5 | 5 | Batch | 2 | 10 | 0.8 | 33.9 |
| N/A | A | 18 | FMM | 5 | 5 | 1 | FMM | 8 | 2 | 0.8 | 11.3 |
| | A | 19 | FMM | 5 | 5 | 1 | FMM | 8 | 2 | 0.8 | 9.2 |
| | A | 20 | Batch | 1 | 1 | 1 | Batch | 0.5 | 2 | 0.2 | 67.1 |
| | B | 21 | Batch | 0.1 | 0.1 | 1 | Batch | 0.05 | 0.2 | 0.2 | 3.6 |
| | B | 22 | FMM | 5 | 5 | 1 | FMM | 2 | 0.5 | 0.8 | 5.3 |
| | B | 23 | FMM | 5 | 5 | 1 | FMM | 4 | 1 | 0.8 | 5.1 |
| | B | 24 | FMM | 5 | 5 | 1 | FMM | 8 | 2 | 0.8 | 4.2 |

**Table 5 (Continued)**

| | | | | | | | | | | | |
|---|---|---|---|---|---|---|---|---|---|---|---|
| 3 | C | 25 | Batch | 1 | 1 | 1 | Batch | 0.05 | 0.2 | 0.2 | 17.0 |
| | C | 26 | FMM | 5 | 5 | 1 | Batch | 2 | 0.5 | 0.8 | 11.2 |
| | C | 27 | Batch | 2 | 2 | 1 | FMM | 8 | 2 | 0.8 | 8.0 |
| | C | 28 | FMM | 5 | 5 | 1 | FMM | 8 | 2 | 0.8 | 6.3 |

### 3. Comparison of Amount of DNA-encapsulated Ferritin Generated by FMM/Batch Examples 1 to 2 (DNA-encapsulated FMM Examples, 1 g/L of ferritin)

Ferritin was disassembled by mixing a 10 mM Tris buffer solution a containing 2 g/L of ferritin and 10 µM of ssDNA (trade name "K3 Et-Free", produced by GeneDesign Inc.) with the solution b to adjust the pH to about 2.3 to 2.5 by using the V-shaped mixer having an inner diameter of 500 µm. In this way, a disassembled solution c having a ferritin concentration of 1 g/L, a dsDNA concentration of 5 µM, and a glycine hydrochloride concentration of 50 mM was obtained.

Ferritin was reassembled by mixing the disassembled solution c and the solution d at predetermined flow rates to adjust the pH to about 7.3 by using the V-shaped mixer having an inner diameter of 500 µm. The flow rates of the solution c and the solution d as well as the concentrations of dsDNA in the reassemblies are shown in Table 6.

**Table 6**

| | Ferritin disassembled solution c | 350 mM Tris hydrochloride buffer solution d | dsDNA encapsulation Concentration |
|---|---|---|---|
| | [mL/min] | [mL/min] | [µM] |
| Example 1 | 8 | 2 | 0.07 |
| Example 2 | 13.5 | 3.4 | 0.07 |

### Comparative Example 1 (DNA-encapsulated Batch Comparative Example, 1 g/L of ferritin, 200 µL)

Ferritin was disassembled by a batch method in which 200 µL of a 10 mM Tris buffer solution a containing 2 g/L of ferritin and 10 µM of ssDNA (trade name "K3 Et-Free", produced by GeneDesign Inc.) was placed in an Eppendorf tube, and 200 µL of the solution b was added and mixed thereto to adjust the pH to about 2.3 to 2.5. In this way, a disassembled solution c having a ferritin concentration of 1 g/L and a dsDNA concentration of 5 µM was obtained.

Ferritin was reassembled by a batch method in which 400 µL of the disassembled solution c was placed in an Eppendorf tube, and 100 µL of the solution d was mixed therewith to adjust the pH to about 7.3. The concentration of DNA encapsulated in ferritin was measured to be less than the quantification limit (0.06 µM).

### Comparative Example 2 (DNA-encapsulated Batch Comparative Example, 1 g/L of ferritin, 2 mL)

Ferritin was disassembled by a batch method in which 1 mL of a 10 mM Tris buffer solution a containing 2 g/L of ferritin and 10 µM of ssDNA (trade name "K3 Et-Free", Produced by GeneDesign Inc.) was placed in a Falcon tube, and 1 mL of the solution b was added and mixed to adjust the pH to about 2.3 to 2.5. In this way, a disassembled solution c having a ferritin concentration of 1 g/L and a dsDNA concentration of 5 µM was obtained.

Ferritin was reassembled by a batch method in which 400 µL of the disassembled solution c was placed in an Eppendorf tube, and 500 µL of the solution d was mixed therewith to adjust the pH to about 7.3. The concentration of DNA encapsulated in ferritin was measure to be less than the quantification limit (0.06 µM).

### Comparison of Amount of siRNA-encapsulated Ferritin Generated by FMM/Batch

### Example 3 (siRNA-encapsulated FMM Example, 1 g/L of ferritin)

Ferritin was disassembled by mixing a 10 mM Tris buffer solution a containing 2 g/L of ferritin and 50 µM of siRNA (produced by EUROFINS GENOMICS, having a base sequence of SEQ ID NO: 1 (GGCGCUGCCAAGGCUGUGGGCAAGGUC)) with the solution b to adjust the pH to about 2.3 to 2.5 by using the V-shaped mixer having an inner diameter of 500 µm. In this way, a disassembled solution c having a ferritin concentration of 1 g/L, a siRNA concentration of 10 µM, and a glycine hydrochloride concentration of 50 mM was obtained.

Ferritin was reassembled by mixing the disassembled solution c and the solution d at predetermined flow rates to adjust the pH to about 7.3 by using the V-shaped mixer having an inner diameter of 500 µm. The flow rates of the solution c and the solution d as well as the concentration of siRNA in the reassemblies obtained in the above analysis are shown in Table 7. Note that in the present specification, "one FTH" indicates a 24-mer of the subunit of FTH, and has the same meaning as 1 mol of FTH.

### Comparative Example 3 (siRNA-encapsulated Batch Comparative Example, 1 g/L of ferritin, 1000 µL)

Ferritin was disassembled by a batch method in which 400 µL of a 10 mM Tris buffer solution a containing 2 g/L of ferritin and 50 µM of siRNA (produced by EUROFINS GENOMICS, having a base sequence of SEQ ID NO: 1) was placed in an Eppendorf tube, and 400 µL of the solution b was added and mixed thereto to adjust the pH to about 2.3 to 2.5. In this way, a disassembled solution c having a ferritin concentration of 1 g/L and a siRNA concentration of 10 µM was obtained.

Ferritin was reassembled by a batch method in which 800 µL of the disassembled solution c was placed in an Eppendorf tube, and 200 µL of the solution d was mixed thereto to adjust the pH to about 7.3. The concentration of siRNA encapsulated in ferritin was measured. The result is shown in Table 7.

**Table 7**

| | Ferritin disassembled solution c | 350 mM Tris hydrochloride buffer solution d | FTH recovery proportion | Aggregate Proportion | Encapsulated amount per one FTH |
|---|---|---|---|---|---|
| | [mL/min] | [mL/min] | (%) | (%) | (mol/mol) |
| Comparative Example 3 (Batch)_1mL scale | - | - | 59.5 | 0.0 | 0.8 |
| Example 3 (FMM) | 8 | 2 | 69.8 | 0.0 | 1.5 |

### Example 4 (siRNA-encapsulated FMM Example, 1 g/L of ferritin)

Ferritin was disassembled by mixing a 10 mM Tris buffer solution a containing 2 g/L of ferritin and 50 µM of siRNA (Produced by Thermo Fisher Scientific, having a base sequence of SEQ ID NO: 2 (GACCUUGCCCACAGCCUUGGCAGCGUC)) with the solution b to adjust the pH to about 2.3 to 2.5 by using the V-shaped mixer having an inner diameter of 500 µm. In this way, a disassembled solution c having a ferritin concentration of 1 g/L, a siRNA concentration of 10 µM, and a glycine hydrochloride concentration of 50 mM was obtained.

Ferritin was reassembled by mixing the disassembled solution c and the solution d at predetermined flow rates to adjust the pH to about 7.3 by using the V-shaped mixer having an inner diameter of 500 µm. The flow rates of the solution c and the solution d as well as the concentration of siRNA in the reassemblies obtained by the above analysis are shown in Table 8.

### Comparative Example 4 (siRNA-encapsulated Batch Comparative Example, 1 g/L of ferritin, 1000 µL)

Ferritin was disassembled by a batch method in which 400 µL of a 10 mM Tris buffer solution a containing 2 g/L of ferritin and 50 µM of siRNA (Produced by Thermo Fisher Scientific, having a base sequence of SEQ ID NO: 2) was placed in an Eppendorf tube, and 400 µL of the solution b was added and mixed thereto to adjust the pH to about 2.3 to 2.5. In this way, a disassembled solution c having a ferritin concentration of 1 g/L and a siRNA concentration of 10 µM was obtained.

Ferritin was reassembled by a batch method in which 800 µL of the disassembled solution c was placed in an Eppendorf tube, and 200 µL of the solution d was mixed thereto to adjust the pH to about 7.3. The concentration of siRNA encapsulated in ferritin was measured. The result is shown in Table 8.

**Table 8**

| | Ferritin disassembled solution c | 350 mM Tris hydrochloride buffer solution d | FTH recovery proportion | Aggregate Proportion | Encapsulated amount per one FTH |
|---|---|---|---|---|---|
| | [mL/min] | [mL/min] | (%) | (%) | (mol/mol) |
| Comparative Example 4 (Batch)_1mL scale | - | - | 63.8 | 6.3 | 0.6 |
| Example 4 (FMM) | 8 | 2 | 79.4 | 3.2 | 1.3 |

### Comparison of Amount of Fluorescent Peptide-encapsulated Ferritin Generated by FMM/Batch

### Example 5 (FAM-venepeptide-encapsulated FMM Example, 1 g/L of ferritin)

Ferritin was disassembled by mixing a 10 mM Tris buffer solution a containing 2 g/L of ferritin and 0.2 mM of FAM-venepeptide (produced by EUROFINS GENOMICS, obtained by fluorescently labeling an amino acid sequence of SEQ ID NO: 3 with fluorescein (FAM-MNVITNLLAGVVHFLGWLV). The fluorescent label is represented by "FAM". The same applies hereinafter.) with the solution b to adjust the pH to about 2.3 to 2.5 by using the V-shaped mixer having an inner diameter of 500 µm. In this way, a disassembled solution c having a ferritin concentration of 1 g/L, a FAM-venepeptide concentration of 62.5 µM, and a glycine hydrochloride concentration of 50 mM was obtained.

Ferritin was reassembled by mixing the disassembled solution c and the solution d at predetermined flow rates to adjust the pH to about 7.3 by using the V-shaped mixer having an inner diameter of 500 µm. The flow rates of the solution c and the solution d as well as the concentration of FAM-venepeptide in the reassemblies obtained by the above analysis are shown in Table 9.

### Comparative Example 5 (FAM-venepeptide-encapsulated Batch Comparative Example, 1 g/L of ferritin, 1000 µL)

Ferritin was disassembled by a batch method in which 400 µL of a 10 mM Tris buffer solution a containing 2 g/L of ferritin and 0.2 mM of FAM-venepeptide (produced by EUROFINS GENOMICS, obtained by fluorescently labeling an amino acid sequence of SEQ ID NO: 3 with fluorescein) was placed in an Eppendorf tube, and 400 µL of the solution b was added and mixed thereto to adjust the pH to about 2.3 to 2.5. In this way, a disassembled solution c having a ferritin concentration of 1 g/L and a FAM-venepeptide concentration of 125 µM was obtained.

Ferritin was reassembled by a batch method in which 800 µL of the disassembled solution c was placed in an Eppendorf tube, and 200 µL of the solution d was mixed thereto to adjust the pH to about 7.3. The concentration of FAM-venepeptide encapsulated in ferritin was measured. The result is shown in Table 9.

**Table 9**

| | Ferritin disassembled solution c | 350 mM Tris hydrochloride buffer solution d | FTH recovery proportion | Aggregate Proportion | Encapsulated amount per one FTH |
|---|---|---|---|---|---|
| | [mL/min] | [mL/min] | (%) | (%) | (mol/mol) |
| Comparative Example 5 (Batch)_1mL scale | - | - | 43.1 | 0.0 | 5 |
| Example 5 (FMM) | 8 | 2 | 58.2 | 0.0 | 12 |

### Comparison of Amount of Fluorescent Peptide-encapsulated Ferritin Generated by FMM/Batch

### Example 6 (FAM-GFIL8-encapsulated FMM Example, 1 g/L of ferritin)

Ferritin was disassembled by mixing a 10 mM Tris buffer solution a containing 2 g/L of ferritin and 1.0 mM of FAM-GFIL8 (produced by EUROFINS GENOMICS, obtained by fluorescently labeling an amino acid sequence of SEQ ID NO: 4 (GFILGFIL) with fluorescein) with the solution b to adjust the pH to about 2.3 to 2.5 by using the V-shaped mixer having an inner diameter of 500 µm. In this way, a disassembled solution c having a ferritin concentration of 1 g/L, a FAM-GFIL8 concentration of 62.5 µM, and a glycine hydrochloride concentration of 50 mM was obtained.

Ferritin was reassembled by mixing the disassembled solution c and the solution d at predetermined flow rates to adjust the pH to about 7.3 by using the V-shaped mixer having an inner diameter of 500 µm. The flow rates of the solution c and the solution d as well as the concentration of FAM-GFIL8 in the reassemblies obtained by the above analysis are shown in Table 10.

### Comparative Example 6 (FAM-GFIL8-encapsulated Batch Comparative Example, 1 g/L of ferritin, 1000 µL)

Ferritin was disassembled by a batch method in which 400 µL of a 10 mM Tris buffer solution a containing 2 g/L of ferritin and 1.0 mM of FAM-GFIL8 (produced by EUROFINS GENOMICS, obtained by fluorescently labeling an amino acid sequence of SEQ ID NO: 4 with fluorescein) was placed in an Eppendorf tube, and 400 µL of the solution b was added and mixed thereto to adjust the pH to about 2.3 to 2.5. In this way, a disassembled solution c having a ferritin concentration of 1 g/L and a FAM-GFIL8 concentration of 125 µM was obtained.

Ferritin was reassembled by a batch method in which 800 µL of the disassembled solution c was placed in an Eppendorf tube, and 200 µL of the solution d was mixed thereto to adjust the pH to about 7.3. The concentration of FAM-GFIL8 encapsulated in ferritin was measured. The result is shown in Table 10.

**Table 10**

| | Ferritin disassembled solution c | 350 mM Tris hydrochloride buffer solution d | FTH recovery proportion | Aggregate Proportion | Encapsulated amount per one FTH |
|---|---|---|---|---|---|
| | [mL/min] | [mL/min] | (%) | (%) | (mol/mol) |
| Comparative Example 6 (Batch)_1mL scale | - | - | 70.9 | 0.0 | 8 |
| Example 6 (FMM) | 8 | 2 | 74.3 | 0.0 | 15 |

### [Sequence Listing Free Text]

SEQ ID NO: 1: RNA
SEQ ID NO: 2: RNA
SEQ ID NO: 3: peptide
SEQ ID NO: 4: peptide

## Claims

1. A method for producing a protein having a supramolecular structure in which a bioactive substance is encapsulated, comprising:
(I) bringing a subunit of a protein, which forms a supramolecular structure, a bioactive substance, and a solution for forming the protein having the supramolecular structure from the subunit into contact with one another in a flow micro mixer.

2. The production method according to claim 1, wherein
the protein having the supramolecular structure is ferritin.

3. The production method according to claim 1 or 2, wherein
in the step (I), a sum of a flow rate of the subunit and a flow rate of the solution is about 10 mL/min or more.

4. The production method according to any one of claims 1 to 3, wherein
the subunit is obtained by (II-1) changing pH of a solution containing the protein having the supramolecular structure to an acidic property or a basic property.

5. The production method according to claim 4, wherein
the solution used in the step (II-1) is a solution having pH of about 1.5 to about 3.0 or pH of about 10 to 12.

6. The production method according to any one of claims 1 to 5, wherein
the solution used in the step (I) for forming the protein having the supramolecular structure from the subunit is a solution having pH of about 5.0 to about 9.0.

7. The production method according to any one of claims 1 to 3, wherein
the subunit is obtained by (II-2) adding a solvent to a solution containing the protein having the supramolecular structure.

8. The production method according to claim 7, wherein
the solvent used in the step (II-2) is selected from the group consisting of dimethyl sulfoxide (DMSO), N,N-dimethylformamide (DMF), acetonitrile, and ethanol.

9. The production method according to claim 7 or 8, wherein
the solution used in the step (I) is selected from the group consisting of a Tris buffer solution, a HEPES buffer solution, a phosphate buffer solution, a boric acid buffer solution, a citric acid buffer solution, a carbonic acid buffer solution, and a glycine buffer solution.

10. The production method according to any one of claims 4 to 9, wherein
the step (II-1) or (II-2) are conducted by using a flow micro mixer.

11. The production method according to any one of claims 1 to 10, wherein
the bioactive substance is selected from the group consisting of siRNAs, DNAs, oligopeptides each having a mass average molecular weight of about 1,000 to about 20,000, or combinations of these.

12. The production method according to any one of claims 4 to 5, wherein
the step (II-1) is conducted by adjusting the pH of the solution of the protein assemblies in which the bioactive substance is not encapsulated, to about 2.3 to about 2.5 by using a glycine hydrochloride buffer having pH of about 2 to about 2.5, and the method further comprises step (I-1) wherein the step (I-1) is conducted by making the pH of the subunit solution of the disassembled protein neutral by using a Tris hydrochloride buffer having pH of about 7.0 to about 9.0.

13. The production method according to claim 12, wherein
the sum of the flow rate of the subunit solution and the flow rate of the Tris hydrochloride buffer is about 10 mL/min or more.

14. The production method according to claim 12 or 13, wherein
the protein is ferritin and the bioactive substance is DNA.

## Patentansprüche

1. Verfahren zur Herstellung eines Proteins mit einer supramolekularen Struktur, in der eine bioaktive Substanz verkapselt ist, umfassend:
(I) In-Kontakt-Bringen einer Untereinheit eines Proteins, das eine supramolekulare Struktur bildet, einer bioaktiven Substanz und einer Lösung zur Bildung des Proteins mit der supramolekularen Struktur aus der Untereinheit miteinander in einem Durchflussmikromischer.

2. Herstellungsverfahren nach Anspruch 1, wobei
das Protein mit der supramolekularen Struktur Ferritin ist.

3. Herstellungsverfahren nach Anspruch 1 oder 2, wobei
in Schritt (I) die Summe aus der Strömungsrate der Untereinheit und der Strömungsrate der Lösung etwa 10 ml/min oder mehr beträgt.

4. Herstellungsverfahren nach einem der Ansprüche 1 bis 3, wobei
die Untereinheit durch (II-1) Ändern des pH einer Lösung, die das Protein mit der supramolekularen Struktur enthält, zu einer sauren Eigenschaft oder einer basischen Eigenschaft erhalten wird.

5. Herstellungsverfahren nach Anspruch 4, wobei
die in Schritt (II-1) verwendete Lösung eine Lösung mit einem pH von etwa 1,5 bis etwa 3,0 oder einem pH von etwa 10 bis 12 ist.

6. Herstellungsverfahren nach einem der Ansprüche 1 bis 5, wobei
die in Schritt (I) verwendete Lösung zur Bildung des Proteins mit der supramolekularen Struktur aus der Untereinheit eine Lösung mit einem pH von etwa 5,0 bis etwa 9,0 ist.

7. Herstellungsverfahren nach einem der Ansprüche 1 bis 3, wobei
die Untereinheit durch (II-2) Zugeben eines Lösungsmittels zu einer Lösung, die das Protein mit der supramolekularen Struktur enthält, erhalten wird.

8. Herstellungsverfahren nach Anspruch 7, wobei
das in Schritt (II-2) verwendete Lösungsmittel aus der aus Dimethylsulfoxid (DMSO), N,N-Dimethylformamid (DMF), Acetonitril und Ethanol bestehenden Gruppe ausgewählt ist.

9. Herstellungsverfahren nach Anspruch 7 oder 8, wobei
die in Schritt (I) verwendete Lösung aus der aus einer Tris-Pufferlösung, einer HEPES-Pufferlösung, einer Phosphat-Pufferlösung, einer Borsäure-Pufferlösung, einer Citronensäure-Pufferlösung, einer Carbonsäure-Pufferlösung und einer Glycin-Pufferlösung bestehenden Gruppe ausgewählt ist.

10. Herstellungsverfahren nach einem der Ansprüche 4 bis 9, wobei
Schritt (II-1) oder (II-2) durch Verwendung eines Durchflussmikromischers ausgeführt wird.

11. Herstellungsverfahren nach einem der Ansprüche 1 bis 10, wobei
die bioaktive Substanz aus der aus siRNAs, DNAs, Oligopeptiden, jeweils mit einem massenmittleren Molekulargewicht von etwa 1.000 bis etwa 20.000, oder Kombinationen davon bestehenden Gruppe ausgewählt ist.

12. Herstellungsverfahren nach einem der Ansprüche 4 bis 5, wobei
Schritt (II-1) durch Einstellen des pH der Lösung der Proteinanordnungen, in denen die bioaktive Substanz nicht verkapselt ist, auf etwa 2,3 bis etwa 2,5 durch Verwendung eines Glycinhydrochloridpuffers mit einem pH von etwa 2 bis etwa 2,5 ausgeführt wird und das Verfahren weiters Schritt (I-1) umfasst, wobei Schritt (I-1) durch Neutralstellen des pH der Untereinheitenlösung des zerlegten Proteins unter Verwendung eines Tris-Hydrochloridpuffers mit einem pH von etwa 7,0 bis etwa 9,0 ausgeführt wird.

13. Herstellungsverfahren nach Anspruch 12, wobei
die Summe aus der Strömungsrate der Untereinheitenlösung und der Strömungsrate des Tris-Hydrochloridpuffers etwa 10 ml/min oder mehr beträgt.

14. Herstellungsverfahren nach Anspruch 12 oder 13, wobei
das Protein Ferritin ist und die bioaktive Substanz DNA ist.

## Revendications

1. Procédé de production d'une protéine ayant une structure supramoléculaire dans laquelle une substance bioactive est encapsulée, comprenant l'étape consistant à :
(I) mettre en contact une sous-unité d'une protéine, qui forme une structure supramoléculaire, une substance bioactive, et une solution pour former la protéine présentant la structure supramoléculaire à partir de la sous-unité en contact les unes avec les autres dans un micro-mélangeur à écoulement.

2. Procédé de production selon la revendication 1, dans lequel
la protéine présentant la structure supramoléculaire est la ferritine.

3. Procédé de production selon la revendication 1 ou 2, dans lequel
dans l'étape (I), une somme d'un débit d'écoulement de la sous-unité et d'un débit d'écoulement de la solution est d'environ 10 mL/min ou plus.

4. Procédé de production selon l'une quelconque des revendications 1 à 3, dans lequel
la sous-unité est obtenue en (II-1) changeant le pH d'une solution contenant la protéine ayant la structure supramoléculaire en une propriété acide ou une propriété basique.

5. Procédé de production selon la revendication 4, dans lequel
la solution utilisée dans l'étape (II-1) est une solution présentant un pH d'environ 1,5 à environ 3,0 ou un pH d'environ 10 à 12.

6. Procédé de production selon l'une quelconque des revendications 1 à 5, dans lequel
la solution utilisée dans l'étape (I) pour former la protéine présentant la structure supramoléculaire à partir de la sous-unité est une solution présentant un pH d'environ 5,0 à environ 9,0.

7. Procédé de production selon l'une quelconque des revendications 1 à 3, dans lequel
la sous-unité est obtenue en (II-2) ajoutant un solvant à une solution contenant la protéine présentant la structure supramoléculaire.

8. Procédé de production selon la revendication 7, dans lequel
le solvant utilisé dans l'étape (II-2) est choisi dans le groupe comprenant le diméthylsulfoxyde (DMSO), le N,N-diméthylformamide (DMF), l'acétonitrile et l'éthanol.

9. Procédé de production selon la revendication 7 ou 8, dans lequel
la solution utilisée dans l'étape (I) est sélectionnée dans le groupe constitué d'une solution tampon Tris, d'une solution tampon HEPES, d'une solution tampon de phosphate, d'une solution tampon d'acide borique, d'une solution tampon d'acide citrique, d'une solution tampon d'acide carbonique et d'une solution tampon de glycine.

10. Procédé de production selon l'une quelconque des revendications 4 à 9, dans lequel
l'étape (II-1) ou (II-2) est effectuée en utilisant un micro-mélangeur à écoulement.

11. Procédé de production selon l'une quelconque des revendications 1 à 10, dans lequel
la substance bioactive est choisie dans le groupe comprenant des ARNsi, des ADN, des oligopeptides présentant chacun un poids moléculaire moyen en masse d'environ 1 000 à environ 20 000, ou des combinaisons de ceux-ci.

12. Procédé de production selon l'une quelconque des revendications 4 à 5, dans lequel
l'étape (II-1) est effectuée en ajustant le pH de la solution des ensembles protéiques dans lesquels la substance bioactive n'est pas encapsulée, entre environ 2,3 et environ 2,5 en utilisant un tampon de chlorhydrate de glycine présentant un pH d'environ 2 à environ 2,5, et le procédé comprend en outre l'étape (I-1), dans lequel l'étape (I-1) est effectuée en rendant le pH de la solution de sous-unité de la protéine désassemblée neutre en utilisant un tampon de chlorhydrate de Tris présentant un pH d'environ 7,0 à environ 9,0.

13. Procédé de production selon la revendication 12, dans lequel
la somme du débit d'écoulement de la solution de sous-unité et du débit d'écoulement du tampon de chlorhydrate de Tris est d'environ 10 mL/min ou plus.

14. Procédé de production selon la revendication 12 ou 13, dans lequel
la protéine est la ferritine et la substance bioactive est l'ADN.
